# EUROPEAN PATENT SPECIFICATION

(11) **EP 4 084 745 B1**
(45) Date of publication and mention of the grant of the patent: **30.10.2024**
(21) Application number: 20848911.2
(22) Date of filing: 30.12.2020
(51) Int. Cl.: A61F 5/44, A61F 5/451

(54) **URINE COLLECTION SYSTEM INCLUDING A SIZING DEVICE**
URINSAMMELSYSTEM, DAS EINE AUSLEGUNGSVORRICHTUNG EINSCHLIESST
SYSTÈME DE COLLECTE D'URINE COMPRENANT UN DISPOSITIF DE DIMENSIONNEMENT

(30) Priority: 03.01.2020 US 202062956767 P
(43) Date of publication of application: 09.11.2022
(73) Proprietor: Purewick Corporation, El Cajon, California 92020 (US)
(72) Inventor: MYERS, Benjamin, Atlanta, GA 30308 (US)
(74) Representative: Hoffmann Eitle
(86) International application number: PCT/US2020/067451
(87) International publication number: WO 2021/138411

(56) References cited:
- EP-A1- 3 424 471
- WO-A1-2019/212952
- US-A- 4 194 508
- US-A- 4 886 508
- US-A1- 2016 374 848
- US-A1- 2018 228 642
- US-A1- 2019 365 307

## Description

### BACKGROUND

An individual may have limited or impaired mobility such that typical urination processes are challenging or impossible. For example, the individual may have surgery or a disability that impairs mobility. In another example, the individual may have restricted travel conditions such as those experienced by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bed pans and urinary catheters have several problems associated therewith. For example, bed pans may be prone to discomfort, spills, and other hygiene issues. Urinary catheters be may be uncomfortable, painful, and may cause urinary tract infections.

Thus, users and manufacturers of urine collection devices continue to seek new and improved devices, systems, and methods to collect urine. The features of the first part of the claim below are disclosed in combination in WO 2019/212952 A1.

### SUMMARY

Claim 1 below defines the invention. The dependent claims are directed to optional features and preferred embodiments. Disclosed herein are urine collection devices and methods of using urine collection devices. Disclosed is a sizing device for such a urine collection system includes a body and a recess. The body has a distal end and an attachment end spaced from the distal end, with the attachment end defining an opening. The recess extends into the body from the opening, with the recess sized and dimensioned to receive a portion of the urine collection device and secure the sizing device to the urine collection device.

Thus, a urine collection system includes a urine collection device and a sizing device. The sizing device is secured or securable to the urine collection device and includes a body and a recess. The body has a distal end and an attachment end spaced from the distal end, with the attachment end defining an opening. The recess extends into the body from the opening, with the recess being sized and dimensioned to receive a portion of the urine collection device and secure the sizing device to the urine collection device.

A method of positioning a urine collection system on an individual or patient is disclosed. The method includes positioning a urine collection device between legs of the individual. The method also includes determining whether securing a sizing device to the urine collection device will improve securement of the urine collection device between the legs of the individual. The method also includes securing the sizing device to a distal portion of the urine collection device. The method also includes positioning the urine collection device with the sizing device secured thereto between the legs of the individual.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present invention, wherein identical reference numerals refer to identical or similar elements or features in different views or embodiments shown in the drawings.
**FIG. 1** is an isometric view of a urine collection system including a sizing device, according to an embodiment.
**FIG. 2** is an isometric view of a urine collection system including a sizing device, according to an embodiment.
**FIG. 3** is an isometric view of a urine collection system including a sizing device, according to an embodiment.
**FIG. 4** is a block diagram of a urine collection system.
**FIG. 5** is a flow diagram of a method for positioning a urine collection device on an individual.

### DETAILED DESCRIPTION

The disclosure which follows relates to urine collection systems and methods of using the same. The urine collection devices and systems disclosed herein are configured to collect fluids from an individual. The fluids collected by the urine collection devices may include at least one of urine, vaginal discharge, penile discharge, reproductive fluids, blood, sweat, or other bodily fluids. The urine collection systems can include a fluid storage container, and a portable vacuum source. The sizing device may be used to more securely position the urine collection device between the legs of an individual using the fluid collection device.

Conventional urine collection devices typically have a size and shape that cannot by modified. Thus, while patients using the urine collection devices have physical or anatomical features that differ in size, geometry, etc., from one patient to another, conventional urine collection devices are not configured to adapt in size and geometry for the varying size and geometry of the physical features of individual patients. This lack of adaptability of the urine collection devices may result in urine collection devices that do not efficiently secure in the desired position between the legs of the user. For example, in a significantly underweight patient, the gap between the legs may be significantly wider than a width of a conventional urine collection device, leaving the conventional urine collection device with a high degree of mobility between the legs and increasing the likelihood of the urine collection device moving from the desired position. In another example, a conventional urine collection device may not be long enough to have a portion of the urine collection device secured at least partially between the buttocks of the patient.

To improve securement of a urine collection device between the legs of a patient using the urine collection device, embodiments of urine collections systems described herein includes a sizing device. As shall be described in greater detail below, the sizing device provides an increased width and length to improve securement of the urine collection device in a desired positioned between the legs of the patient using the urine collection device. The sizing device may be removably secured to the urine collection device. For example, the sizing device may include a body and a recess in the body that is sized and shaped complementary to a distal portion of the urine collection device to allow a distal portion of the urine collection device to be secured within the recess on the urine collection device. The sizing device may include one or more of a substantially conical shape, a wedge shape, a bulbous shape, a disc shape, or other suitable shapes. The sizing devices described herein may be detached from the urine collection device after use, thereby allowing the sizing device to be cleaned and reused.

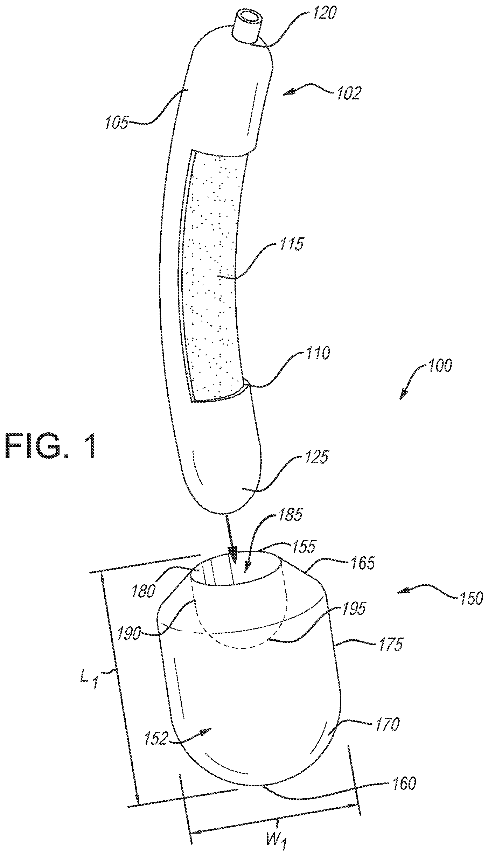

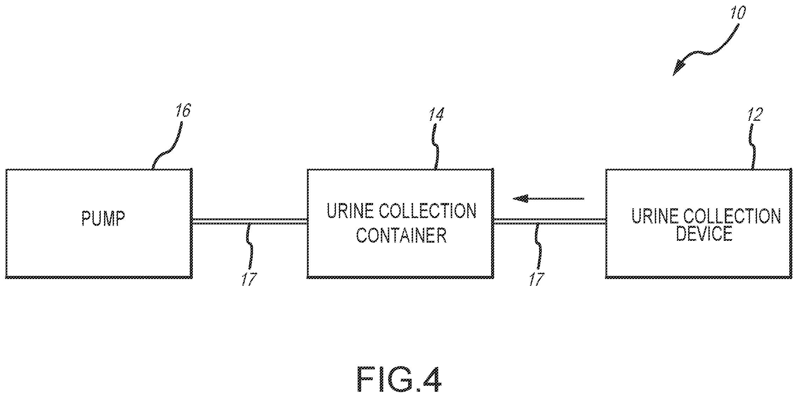

**FIG. 1** is an isometric view of a urine collection device 102 and a sizing device 150 of a urine collection system 100. Although not shown in **FIG. 1**, the urine collection system 100 also may include a fluid storage container and a portable vacuum source, as described below in the block diagram of **FIG. 4****.** The urine collection device 102 may include a fluid impermeable barrier 105 having an end portion 125 and at least partially defining a chamber, an aperture 120 distal to the end portion 125 and configured to receive a conduit therethrough, and an opening 110 between the aperture 120 and the end portion 125. A fluid permeable support 115 may be positioned at least partially within the chamber defined by the fluid impermeable barrier 105 to extend across at least a portion of the opening 110. The fluid permeable support 115 may be configured to wick fluid from the opening 110 into the chamber for removal from the urine collection device. The fluid permeable support 115 includes an outer layer of a fluid permeable membrane including gauze and an inner support of a porous nylon structure.

The fluid impermeable barrier 105 may be generally tubular or cylindrical. Accordingly, the end portion 125 of the fluid impermeable barrier 105 may be generally tubular. For example, in the urine collection device 102 shown in **FIG. 1**, the end portion 125 is a tapered end portion having a cylindrical portion a rounded or semispherical portion. While the end portion 125 forms a portion of the fluid impermeable barrier 105 of the urine collection device 102, in other embodiments, the end portion 125 may be secured to the fluid impermeable barrier 105 and may include a different material than the fluid impermeable barrier 105. Other embodiments of urine collection devices including fluid impermeable barriers, fluid permeable supports, chambers, and their shapes and configurations are disclosed in U.S. Patent Application No. 15/260,103 filed on September 8, 2016 and U.S. Patent Application No. 15/611,587 filed on June 1, 2017.

The sizing device 150 is configured to be detachably secured to the urine collection device 102 at the end portion 125 of the urine collection device 102. The sizing device 150 may be secured to the urine collection device 102 for use with a patient, then removed from the urine collection device 102, cleaned, and reused on a different urine collection device 102 and/or with a different patient. The sizing device 150 is sized and dimensioned to provide both additional length and width to the urine collection device 102.

The sizing device 150 includes a body 152 and a recess 185. The body 152 may be formed from a biocompatible material that will not cause excess friction, harm, or discomfort to the patient using the urine collection device 102 and the sizing device 150. For example, the body 152 may include one or more of a biocompatible foam, silicone, latex rubber, or other suitable material. In some embodiments, the body 152 includes substantially the same material as the fluid impermeable membrane 105 of the urine collection device 105. The body 152 of the sizing device 150 may be substantially hollow and filled with air. In some embodiments, the body 152 may be filled with a relatively soft or pliable material, or may be filled with the same biocompatible material forming the outer surface of the body 152.

The body 152 of the sizing device 150 includes a head 165, a base 170, an attachment end 155 at the head 165, an opening 180 defined by the attachment end 155 and/or the head 165, and a distal end 160 at the base 170. The head 165 may be substantially frustoconical and taper to the attachment end 155, and the base 170 may be substantially semispherical and taper to the distal end 160 of the body 152. The body 152 also may include a substantially cylindrical portion 175 between the head 165 and the base 170. In some embodiments, the head 165 tapers from the cylindrical portion 175 to the opening 180 at the attachment end 155 to form the substantially frustoconical shape of the head 165. The head 165 may taper continuously or only partially between the cylindrical portion 175 and the attachment end 155. In some embodiments, the base 170 tapers from the cylindrical portion 175 to the distal end 160 to form the substantially semispherical shape of the base 170. The base 170 may taper continuously or only partially between the cylindrical portion 175 and the distal end 160.

The body 152 includes a length L₁ from the attachment end 155 to the distal end 160. The length L₁ may vary according to different embodiments. For example, the length L₁ may be at least about 2.5 cm, at least about 3.8 cm, at least about 5.1 cm, at least about 6.4 cm, at least about 7.6 cm, at least about 8.9 cm, at least about 10.2 cm, about 2.5 cm to about 12.7 cm, about 2.5 cm to about 7.6 cm, about 7.6 cm to about 12.7 cm, about 2.5 cm to about 5.1 cm, about 5.1 cm to about 7.6 cm, about 7.6 cm to about 10.2 cm, about 10.2 cm to about 12.7 cm, about 2.5 cm, about 3.8 cm, about 5.1 cm, about 6.4 cm, about 7.6 cm, about 8.9 cm, or about 10.2 cm.

The body 152 also includes a maximum width W₁ that may, for example, be the diameter of the cylindrical portion 175. The maximum width W₁ may vary according to different embodiments. For example, the maximum width W₁ may be at least about 3.8 cm, at least about 5.1 cm, at least about 6.4 cm, at least about 7.6 cm, at least about 8.9 cm, at least about 10.2 cm, about 2.5 cm to about 12.7 cm, about 3.8 cm to about 7.6 cm, about 7.6 cm to about 12.7 cm, about 3.8 cm to about 5.1 cm, about 5.1 cm to about 7.6 cm, about 7.6 cm to about 10.2 cm, about 10.2 cm to about 12.7 cm, about 3.8 cm, about 5.1 cm, about 6.4 cm, about 7.6 cm, about 8.9 cm, or about 10.2 cm.

The opening 180 may include a width substantially equal to a width of the end portion 125 of the urine collection device 102, such as a diameter of the cylindrical portion of the end portion 125. For example, the opening may include a width or diameter of about 1.3 cm to about 5.1 cm, about 1.3 cm to about 2.5 cm, about 2.5 cm to about 3.8 cm, about 3.8 cm to about 5.1 cm, about 1.3 cm, about 1.9 cm, about 2.5 cm, about 3.2 cm, about 3.8 cm, about 4.4 cm, or about 5.1 cm.

The sizing device 150 also includes the recess 185 extending partially into the body 152 from the opening 180. In the sizing device 150 shown in **FIG. 1**, the recess 185 extends partially into the body 152 from the opening 180 at the distal end 155 of the body 152. The recess 185 may be defined at least partially by an inner surface of the head 165. In some embodiments, the recess 185 is defined at least partially by an inner surface of the head 165 and an inner surface of the cylindrical portion 175. The inner surface(s) defining the recess 185 may include any of the materials described above in relation to the material of the body 152. The inner surface(s) defining the recess 185 additionally or alternatively may include one or more of a hook or loop material (such as VELCRO^{®}), an adhesive, or tape.

The recess 185 is shaped complementary to the end portion 125 of the urine collection device 102. For example, the recess 185 may include a cylindrical portion 190 and a semispherical portion 195 shaped to correspond with part of the cylindrical portion and the semispherical portion of the end portion 125 of the urine collection device 102. The recess 185 may include a maximum width or diameter substantially equal to any of the width described above in relation to the opening 180. The recess 185 also includes a maximum depth from the opening 180 to the bottom of the recess 185. The depth may be about 0.64 cm to about 3.8 cm, about 0.64 cm to about 1.3 cm, about 1.3 cm to about 1.9 cm, about 1.9 cm to about 2.5 cm, about 2.5 cm to about 3.2 cm, about 3.2 cm to about 3.8 cm, at least about 0.64 cm, at least about 1.3 cm, at least about 1.9 cm, at least about 2.5 cm, at least about 3.2 cm, or at least about 3.8 cm. In some embodiments, the recess 185 includes a through hole that passes through the body 152, the through hole being sized to allow at least a portion of the urine collection device 102 to slide through the through hole and protrude from the distal end 160 of the body 152.

The end portion 125 of the urine collection device 102 may be secured within the recess 185 due to one or more of friction from tight tolerances between the end portion 125 and the inner surface(s) defining the recess 185, an elastic material in at least one of the end portion 125 or the inner surface(s) defining recess 185, an interlock system between the end portion 125 and body 152, a hook and loop system (such as VELCRO^{®}), one or more adhesives, tape, clips, or other suitable materials. In use, the end portion 125 of the urine collection device 102 may be inserted into the recess 185 of the sizing device 150 to secure the sizing device 150 to the urine collection device 102. Securing the sizing device 150 to the urine collection device 102 adds increased length and width to the urine collection device 102. With additional length and width added to urine collection device 102, stability and securement of the urine collection device 102 between the legs of the patient using the urine collection device 102 may be improved.

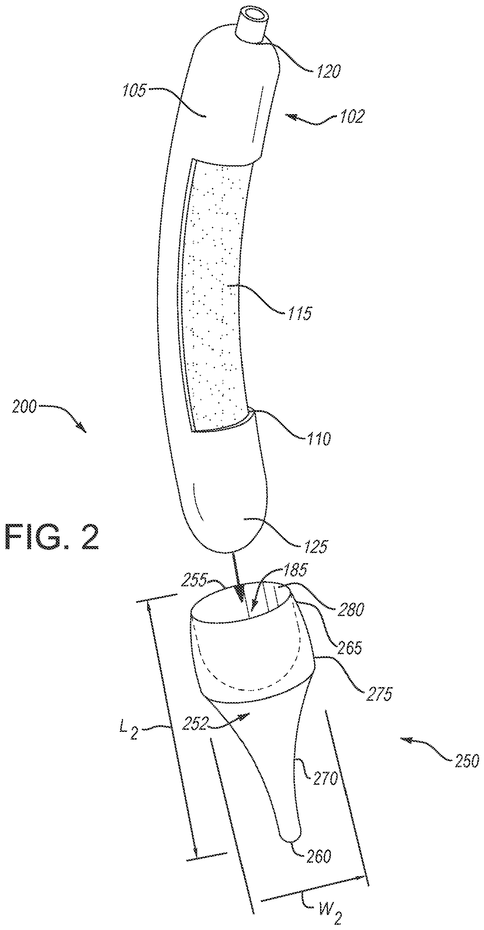

**FIG. 2** is an isometric view of the urine collection device 102 and a sizing device 250 of a urine collection system 200. Although not shown in **FIG. 2**, the urine collection system 200 also may include a fluid storage container and a portable vacuum source, as described above. The sizing device 250 is configured to detachably secure to the urine collection device 102 at the end portion 125 of the urine collection device 102. The sizing device 250 may be secured to the urine collection device 102 for use with a patient, then removed from the urine collection device 102, cleaned, and reused on a different urine collection device 102 and/or with a different patient. The sizing device 250 is sized and dimensioned to provide additional length to the urine collection device 102.

The sizing device 250 includes a body 252 and the recess 185. The recess 185 may include any of the features, dimensions, and characteristics described above in relation to the sizing device 150. The body 252 may be formed from any of the materials described above in relation to the body 152 of the sizing device 150.

The body 252 of the sizing device 250 includes a head 265, a base 270, an attachment end 255 at the head 265, an opening 280 defined by the attachment end 255 and/or the head 265, and a distal end 260 at the base 270. The head 265 may be at least partially frustoconical and taper to the attachment end 255, and the base 270 may be an elongated conical or frustoconical shape that tapers to the distal end 260 of the body 252. More particularly, the base 270 may have generally inward curved or concave sides as the base tapers to the distal end 260. The body 252 also may include a substantially cylindrical portion 275 between the head 265 and the base 270. In some embodiments, the head 265 tapers from the cylindrical portion 275 to the opening 280 at the attachment end 255 to form the at least partially frustoconical shape of the head 265. The head 265 may taper continuously or only partially between the cylindrical portion 275 and the attachment end 255. In some embodiments, the base 270 tapers from the cylindrical portion 275 to the distal end 260 to form the elongated conical or frustoconical shape of the base 270. The base 270 may taper continuously or only partially between the cylindrical portion 275 and the distal end 260. In other embodiments, the body 252 includes a substantially cylindrical head 265 and the elongated conical or frustoconical base 270.

The body 252 includes a length L₂ from the attachment end 255 to the distal end 260. The length L₂ may vary according to different embodiments. For example, the length L₂ may be at least about 2.5 cm, at least about 3.8 cm, at least about 5.1 cm, at least about 6.4 cm, at least about 7.6 cm, at least about 8.9 cm, at least about 10.2 cm, at least about 11.4 cm, at least about 12.7 cm, about 2.5 cm to about 15.24 cm, about 2.5 cm to about 7.6 cm, about 7.6 cm to about 12.7 cm, about 2.5 cm to about 5.1 cm, about 5.1 cm to about 7.6 cm, about 7.6 cm to about 10.2 cm, about 10.2 cm to about 12.7 cm, about 12.7 cm to about 15.24 cm, about 2.5 cm, about 3.8 cm, about 5.1 cm, about 6.4 cm, about 7.6 cm, about 8.9 cm, about 10.2 cm, about 4.12.7 cm, about 12.7 cm, about 14 cm, or about 15.24 cm.

The body 252 also includes a maximum width W₂ that may, for example, be the diameter of the cylindrical portion 275. The maximum width W₂ may vary according to different embodiments. For example, the maximum width W₂ may be at least about 2.5 cm, at least about 3.2 cm, at least about 3.8 cm, at least about 4.4 cm, at least about 5.1 cm, about 2.5 cm to about 5.1 cm, about 2.5 cm to about 3.2 cm, about 3.2 cm to about 3.8 cm, about 3.8 cm to about 4.4 cm, about 4.4 cm to about 5.1 cm, about 2.5 cm, about 3.2 cm, about 3.8 cm, or about 5.1 cm.

The opening 280 may include a width substantially equal to a width of the end portion 125 of the urine collection device 102, such as a diameter of the cylindrical portion of the end portion 125. For example, the opening 280 may include a width or diameter of about 1.3 cm to about 5.1 cm, about 1.3 cm to about 2.5 cm, about 2.5 cm to about 3.8 cm, about 3.8 cm to about 5.1 cm, about 1.3 cm, about 1.9 cm, about 2.5 cm, about 3.2 cm, about 3.8 cm, about 4.4 cm, or about 5.1 cm.

In use, the end portion 125 of the urine collection device 102 is inserted into the recess 185 of the sizing device 250 to secure the sizing device 250 to the urine collection device 102. Securing the sizing device 250 to the urine collection device 102 adds increased length to the urine collection device 102. With additional length added to the urine collection device 102, stability and securement of the urine collection device 102 between the legs of the patient using the urine collection device 102 may be improved. For example, the length added to the urine collection device 102 may allow at least a portion of the base 270 to be positioned between the buttocks of the patient wearing the urine collection device 102.

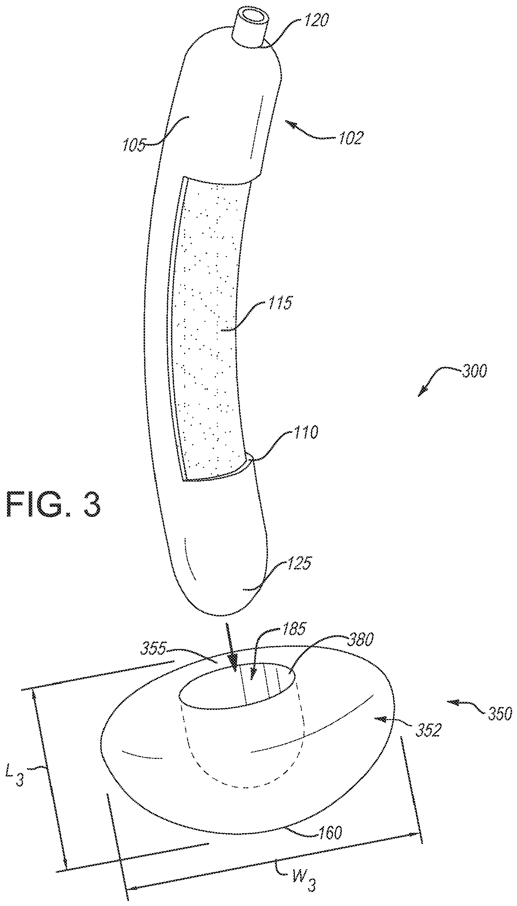

**FIG. 3** is an isometric view of the urine collection device 102 and a sizing device 350 of a urine collection system 300. Although not shown in **FIG. 3**, the urine collection system 300 also may include a fluid storage container and a portable vacuum source, as described above. The sizing device 350 is configured to detachably secure to the urine collection device 102 at the end portion 125 of the urine collection device 102. The sizing device 350 may be secured to the urine collection device 102 for use with a patient, then removed from the urine collection device 102, cleaned, and reused on a different urine collection device 102 and/or with a different patient. The sizing device 350 is sized and dimensioned to provide additional width to the urine collection device 102.

The sizing device 350 includes a body 352 and the recess 185. The recess 185 may include any of the features, dimensions, and characteristics described above in relation to the sizing device 350. The body 352 may be formed from any of the materials described above in relation to the body 152 of the sizing device 150. The body 352 of the sizing device 350 includes an attachment end 355, an opening 380 defined by the attachment end, and a distal end 360 distal to the attachment end 355. The body 352 may be generally ellipsoidal, semispherical, spherical, or bulbous in shape.

The body 352 includes a length L₃ from the attachment end 355 to the distal end 360. The length L₃ may vary according to different embodiments. For example, the length L₃ may be at least about 2.5 cm, at least about 3.8 cm, at least about 5.1 cm, at least about 6.4 cm, at least about 7.6 cm, about 2.5 cm to about 10.2 cm, about 2.5 cm to about 7.6 cm, about 5.1 cm to about 10.2 cm, about 2.5 cm to about 5.1 cm, about 5.1 cm to about 7.6 cm, about 7.6 cm to about 10.2 cm, about 2.5 cm, about 3.8 cm, about 5.1 cm, about 6.4 cm, about 7.6 cm, about 8.9 cm, or about 10.2 cm.

The body 352 also includes a maximum width W₃ that may vary according to different embodiments. For example, the maximum width W₃ may be at least about 5.1 cm, at least about 6.4 cm, at least about 7.6 cm, at least about 8.9 cm, at least about 10.2 cm, about 5.1 cm to about 7.6 cm, about 7.6 cm to about 10.2 cm, about 10.2 cm to about 12.7 cm, about 5.1 cm, about 6.4 cm, about 7.6 cm, about 8.9 cm, or about 10.2 cm.

The opening 380 may include a width substantially equal to a width of the end portion 125 of the urine collection device 102, such as a diameter of the cylindrical portion of the end portion 125. For example, the opening 380 may include a width or diameter of about 1.3 cm to about 5.1 cm, about 1.3 cm to about 2.5 cm, about 2.5 cm to about 3.8 cm, about 3.8 cm to about 5.1 cm, about 1.3 cm, about 1.9 cm, about 2.5 cm, about 3.2 cm, about 3.8 cm, about 4.4 cm, or about 5.1 cm.

The originally filed claims include the following points of disclosure of various dimensions of the urine collection device and/or its associated sizing device:
1. the body of the sizing device can include a length from its distal end to its attachment end of at least 5.1 cm and a maximum width of at least about 3.75 cm
2. the sizing device can have an elongated conical or frustoconical base tapering at least partially between the cylindrical portion and the distal end, which base is at least about 3.75 cm in length
3. the body of the sizing device can include a length from the distal end to the attachment end of at least 6.25 cm and a maximum width of at least about 2.5 cm
4. the body of the sizing device can include a generally ellipsoid shape, the body including a maximum width of at least 6.25 cm
5. the body of the sizing device can include a length of about 2.5 cm to about 5 cm
6. the opening in the sizing device can include a diameter of about 1.9 cm to about 3.175 cm
7. the recess in the sizing device can include a depth of about 1.25 cm to about 2.5 cm.

In use, the end portion 125 of the urine collection device 102 may be inserted into the recess 185 of the sizing device 350 to secure the sizing device 350 to the urine collection device 102. Securing the sizing device 350 to the urine collection device 102 adds increased width to the urine collection device 102. With additional width added to the urine collection device 102, stability and securement of the urine collection device 102 between the legs of the patient using the urine collection device 102 may be improved. For example, the width added to the urine collection device 102 may allow the sides of the sizing device 350 to contact the legs of the patient wearing the urine collection device 102.

**FIG. 4** is a block diagram of a fluid collection system 10 which includes a fluid (*e.g.*, urine) collection device 12 (*e.g.*, any of the fluid collection device disclosed herein), a urine collection container 14, and a pump 16 (or vacuum source). The fluid collection device 12, the urine collection container 14, and the pump 16 may be fluidly coupled to each other via one or more conduits 17. For example, fluid collection device 12 may be operably coupled to one or more of the urine collection container 14 or the pump 16 via the conduit 17. In some embodiments, the pump 16 may be secured directly to the urine collection container 14. Fluid (*e.g*., urine or other bodily fluids) collected in the fluid collection device 12 may be removed from the fluid collection device 12 via the conduit 17 secured to the fluid collection device 12. Suction force may be introduced into the chamber of the fluid collection device 12 via the inlet of the conduit 17 responsive to suction (*e.g*., vacuum) force applied at the outlet of the conduit 17.

The suction force may be applied to the outlet of the conduit 17 by the pump 16 either directly or indirectly. The suction force may be applied indirectly via the urine collection container 14. For example, the outlet of the conduit 17 may be disposed within or fluidly coupled to an interior region of the urine collection container 14 and an additional conduit 17 may extend from the urine collection container 14 to the pump 16. Accordingly, the pump 16 may apply suction to the fluid collection device 12 via the urine collection container 14. The suction force may be applied directly via the pump 16. For example, the outlet of the conduit 17 may be disposed within the pump 16. An additional conduit 17 may extend from the pump 16 to a point outside of the fluid collection device 12, such as to the urine collection container 14. In such examples, the pump 16 may be disposed between the fluid collection device 12 and the urine collection container 14.

The urine collection container 14 is sized and shaped to retain a fluid therein. The urine collection container 14 may include a bag (*e.g*., drainage bag), a bottle or cup (*e.g*., collection jar), or any other enclosed container for storing bodily fluid(s) such as urine. In some examples, the conduit 17 may extend from the fluid collection device 12 and attach to the urine collection container 14 at a first point therein. An additional conduit 17 may attach to the urine collection container 14 at a second point thereon and may extend and attach to the pump 16. Accordingly, a vacuum (*e.g*., suction) may be drawn through fluid collection device 12 via the urine collection container 14. Fluid, such as urine, may be drained from the fluid collection device 12 using the pump 16.

The pump 16 or vacuum source may include one or more of a manual vacuum pump, and electric vacuum pump, a diaphragm pump, a centrifugal pump, a displacement pump, a magnetically driven pump, a peristaltic pump, or any pump configured to produce a vacuum. The pump 16 may provide a vacuum or suction to remove fluid from the fluid collection device 12. In some examples, the pump 16 may be powered by one or more of a power cord (*e.g*., connected to a power socket), one or more batteries, or even manual power (*e.g*., a hand operated vacuum pump). In some examples, the pump 16 may be sized and shaped to fit outside of, on, or within the fluid collection device 12. For example, the pump 16 may include one or more miniaturized pumps or one or more micro pumps. The vacuum sources disclosed herein may include one or more of a switch, a button, a plug, a remote, or any other device suitable to activate the pump 16.

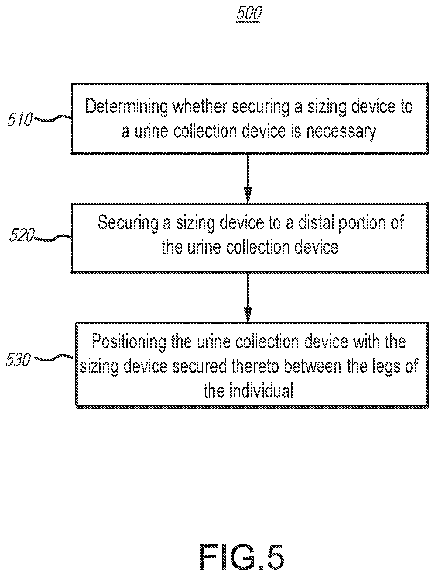

**FIG. 5** is a flow diagram of a method 500 of positioning a urine collection system on an individual. The method 500 may include an act of positioning a urine collection device between legs of the individual. The urine collection device may include any of the urine collection devices described herein, such as the urine collection device 102.

The method 500 may include an act 510 determining whether securing a sizing device to the urine collection device will improve securement of the urine collection device between the legs of the individual. The sizing device may include any of the sizing devices described herein, such as sizing device 100, 200, or 300. The act 510 of determining whether securing a sizing device to the urine collection device will improve securement of the urine collection device between the legs of the individual may include positioning different sizing devices between the legs of the individual to identify a sizing device that interfaces or contacts the legs or gluteal cleft of the individual effective to remain in place between the legs of the individual when the individual is laying down.

The method 500 also may include an act 520 of securing the sizing device to a distal portion of the urine collection device. The act 520 of securing the sizing device to a distal portion of the urine collection device may include inserting the distal portion of the urine collection device into a recess in the sizing device. The method also may include an act 530 of positioning the urine collection device with the sizing device secured thereto between the legs of the individual.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

## Claims

1. A urine collection system (100, 200, 300), which includes an elongate urine collection device (102) having a cylindrical lower end portion (125), an aperture (120) distal to the end portion configured to receive a conduit and an opening (110) between the aperture and the end portion to receive urine into the device
**characterized in that**:
the urine collection system includes a sizing device (150, 250, 350), the sizing device comprising:
a body (152, 252, 352) having a distal base end (160, 260) and an attachment upper end (155, 255, 355) spaced from the distal end, the attachment end defining an upper opening (180, 280, 380); and
a recess (185) including a cylindrical portion (190), the recess extending into the body from the upper opening, the recess being sized and dimensioned to receive the cylindrical lower end portion of the urine collection device and secure the sizing device to the urine collection device, the sizing device being removable from the urine collection device,
the longitudinal axis of the sizing device (150) being coaxial with the longitudinal axis of the end portion (125) of the urine collection device (102) when the end portion is received in the recess (185) of the sizing device, the sizing device having a maximum width greater than a maximum width of the urine collection device and thereby serving, when secured to the lower end of the urine collection device, to provide additional length and width to the lower end of the urine collection device and thereby improve securement of the urine collection device in a desired position between the legs of the person wearing the urine collection device.

2. The system of claim 1, wherein the body (152, 252, 352) includes a substantially cylindrical portion (175, 275) positioned between the attachment end (155, 255, 355) and the distal end (160, 260).

3. The system of claim 2, further comprising a substantially frustoconical head tapering at least partially between the cylindrical portion (190) and the attachment end (155, 255, 355).

4. The system of claim 2 or 3, further comprising a semispherical base (170) tapering at least partially between the cylindrical portion (190) and the distal end (160, 260).

5. The system of any of claims 2 to 4, wherein the body (152, 252, 352) includes a length from the distal end (160, 260) to the attachment end (155, 255, 355) of at least 5.1 cm and a maximum width of at least 3.75 cm.

6. The system of claim 2, further comprising an elongated conical or frustoconical base (170) tapering at least partially between the cylindrical portion (190) and the distal end (160, 260).

7. The system of claim 6, wherein the elongated conical or frustoconical base (170) is at least 3.75 cm in length.

8. The system of claim 6 or 7, wherein the body (152, 252, 352) includes a length from the distal end (160, 260) to the attachment end (155, 255, 355) of at least 6.25 cm and a maximum width of at least 2.5 cm.

9. The system of claim 1, wherein the body (152, 252, 352) includes a generally ellipsoid shape.

10. The system of claim 9, wherein the body (152, 252, 352) includes a maximum width of at least 6.25 cm.

11. The system of claim 9 or 10, wherein the body (152, 252, 352) includes a length of 2.5 cm to 5 cm.

12. The system of any of claims 1 to 11, wherein the collection device-receiving opening (180) includes a diameter of 1.9 cm to 3.175 cm.

13. The system of any of claims 1 to 12, wherein the recess (185) includes a depth of 1.25 cm to 2.5 cm.

14. The system of any of claims 1 to 13, wherein the recess (185) includes a substantially semispherical portion (170).

15. The system as claimed in any one of the preceding claims, and, comprising:
different sizing devices, thereby improving securement of the urine collection device between the legs of the individual wearer by identifying a sizing device from amongst the different sizing devices, which identified sizing device interfaces or contacts the legs or gluteal cleft of the individual wearer in a way effective for the device to remain in place when the wearer is laying down.

## Patentansprüche

1. Urinsammelsystem (100, 200, 300), das eine längliche Urinsammelvorrichtung (102) mit einem zylindrischen unteren Endabschnitt (125), einer Öffnung (120) distal zu dem Endabschnitt, die zur Aufnahme einer Leitung konfiguriert ist, und einer Öffnung (110) zwischen der Öffnung und dem Endabschnitt zur Aufnahme von Urin in die Vorrichtung einschließt,
**dadurch gekennzeichnet, dass**:
das Urinsammelsystem eine Auslegungsvorrichtung (150, 250, 350) einschließt, die Auslegungsvorrichtung umfassend:
einen Körper (152, 252, 352) mit einem distalen Basisende (160, 260) und einem oberen Befestigungsende (155, 255, 355), das von dem distalen Ende beabstandet ist, wobei das Befestigungsende eine obere Öffnung (180, 280, 380) definiert; und
eine Aussparung (185) einschließlich eines zylindrischen Abschnitts (190), wobei sich die Aussparung von der oberen Öffnung in den Körper hinein erstreckt, wobei die Aussparung so bemessen und dimensioniert ist, dass sie den zylindrischen unteren Endabschnitt der Urinsammelvorrichtung aufnimmt und die Auslegungsvorrichtung an der Urinsammelvorrichtung befestigt, wobei die Auslegungsvorrichtung von der Urinsammelvorrichtung abnehmbar ist,
wobei die Längsachse der Auslegungsvorrichtung (150) koaxial mit der Längsachse des Endabschnitts (125) der Urinsammelvorrichtung (102) ist, wenn der Endabschnitt in der Aussparung (185) der Auslegungsvorrichtung aufgenommen ist, wobei die Auslegungsvorrichtung eine maximale Breite aufweist, die größer ist als eine maximale Breite der Urinsammelvorrichtung, und die, wenn sie am unteren Ende der Urinsammelvorrichtung befestigt ist, dazu dient, dem unteren Ende der Urinsammelvorrichtung zusätzliche Länge und Breite zu verleihen, und dadurch die Befestigung der Urinsammelvorrichtung in einer gewünschten Position zwischen den Beinen der Person, die die Urinsammelvorrichtung trägt, verbessert.

2. System nach Anspruch 1, wobei der Körper (152, 252, 352) einen im Wesentlichen zylindrischen Abschnitt (175, 275) einschließt, der zwischen dem Befestigungsende (155, 255, 355) und dem distalen Ende (160, 260) angeordnet ist.

3. System nach Anspruch 2, weiter umfassend einen im Wesentlichen kegelstumpfförmigen Kopf, der sich zumindest teilweise zwischen dem zylindrischen Abschnitt (190) und dem Befestigungsende (155, 255, 355) verjüngt.

4. System nach Anspruch 2 oder 3, weiter umfassend eine halbkugelförmige Basis (170), die sich zumindest teilweise zwischen dem zylindrischen Abschnitt (190) und dem distalen Ende (160, 260) verjüngt.

5. System nach einem der Ansprüche 2 bis 4, wobei der Körper (152, 252, 352) eine Länge vom distalen Ende (160, 260) bis zum Befestigungsende (155, 255, 355) von mindestens 5,1 cm und eine maximale Breite von mindestens 3,75 cm einschließt.

6. System nach Anspruch 2, weiter umfassend eine längliche konische oder kegelstumpfförmige Basis (170), die sich zumindest teilweise zwischen dem zylindrischen Abschnitt (190) und dem distalen Ende (160, 260) verjüngt.

7. System nach Anspruch 6, wobei die längliche konische oder kegelstumpfförmige Basis (170) eine Länge von mindestens 3,75 cm hat.

8. System nach Anspruch 6 oder 7, wobei der Körper (152, 252, 352) eine Länge vom distalen Ende (160, 260) zum Befestigungsende (155, 255, 355) von mindestens 6,25 cm und eine maximale Breite von mindestens 2,5 cm einschließt.

9. System nach Anspruch 1, wobei der Körper (152, 252, 352) im Allgemeinen eine ellipsoide Form einschließt.

10. System nach Anspruch 9, wobei der Körper (152, 252, 352) eine maximale Breite von mindestens 6,25 cm einschließt.

11. System nach Anspruch 9 oder 10, wobei der Körper (152, 252, 352) eine Länge von 2,5 cm bis 5 cm einschließt.

12. System nach einem der Ansprüche 1 bis 11, wobei die Aufnahmeöffnung (180) für die Sammelvorrichtung einen Durchmesser von 1,9 cm bis 3,175 cm einschließt.

13. System nach einem der Ansprüche 1 bis 12, wobei die Aussparung (185) eine Tiefe von 1,25 cm bis 2,5 cm einschließt.

14. System nach einem der Ansprüche 1 bis 13, wobei die Aussparung (185) einen im Wesentlichen halbkugelförmigen Abschnitt (170) einschließt.

15. System nach einem der vorstehenden Ansprüche, und umfassend:
verschiedene Auslegungsvorrichtungen, wodurch die Sicherung der Urinsammelvorrichtung zwischen den Beinen des individuellen Trägers verbessert wird, indem eine Auslegungsvorrichtung unter den verschiedenen Auslegungsvorrichtungen identifiziert wird, wobei die identifizierte Auslegungsvorrichtung mit den Beinen oder der Gesäßspalte des individuellen Trägers in einer Weise in Berührung kommt oder diese berührt, die bewirkt, dass die Vorrichtung an ihrem Platz bleibt, wenn der Träger sich hinlegt.

## Revendications

1. Système de collecte d'urine (100, 200, 300), qui inclut un dispositif de collecte d'urine (102) allongé présentant une partie d'extrémité (125) inférieure cylindrique, une lumière (120) distale par rapport à la partie d'extrémité configurée pour recevoir un conduit et une ouverture (110) entre la lumière et la partie d'extrémité pour recevoir l'urine dans le dispositif
**caractérisé en ce que** :
le système de collecte d'urine inclut un dispositif de dimensionnement (150, 250, 350), le dispositif de dimensionnement comprenant :
un corps (152, 252, 352) présentant une extrémité de base distale (160, 260) et une extrémité supérieure de fixation (155, 255, 355) espacée de l'extrémité distale, l'extrémité de fixation définissant une ouverture supérieure (180, 280, 380) ; et
un évidement (185) incluant une partie cylindrique (190), l'évidement s'étendant dans le corps à partir de l'ouverture supérieure, l'évidement présentant une taille et des dimensions pour recevoir la partie d'extrémité inférieure cylindrique du dispositif de collecte d'urine et fixer le dispositif de dimensionnement au dispositif de collecte d'urine, le dispositif de dimensionnement pouvant être enlevé du dispositif de collecte d'urine,
l'axe longitudinal du dispositif de dimensionnement (150) étant coaxial avec l'axe longitudinal de la partie d'extrémité (125) du dispositif de collecte d'urine (102) lorsque la partie d'extrémité est reçue dans l'évidement (185) du dispositif de dimensionnement, le dispositif de dimensionnement présentant une largeur maximale supérieure à une largeur maximale du dispositif de collecte d'urine et servant ainsi, lorsqu'il est fixé à l'extrémité inférieure du dispositif de collecte d'urine, à fournir une longueur et une largeur additionnelles à l'extrémité inférieure du dispositif de collecte d'urine pour ainsi améliorer la fixation du dispositif de collecte d'urine dans une position souhaitée entre les jambes de la personne portant le dispositif de collecte d'urine.

2. Système selon la revendication 1, dans lequel le corps (152, 252, 352) inclut une partie sensiblement cylindrique (175, 275) positionnée entre l'extrémité de fixation (155, 255, 355) et l'extrémité distale (160, 260).

3. Système selon la revendication 2, comprenant en outre une tête sensiblement tronconique s'étroitisant au moins partiellement entre la partie cylindrique (190) et l'extrémité de fixation (155, 255, 355).

4. Système selon la revendication 2 ou la revendication 3, comprenant en outre une base (170) semi-sphérique s'étroitisant au moins partiellement entre la partie cylindrique (190) et l'extrémité distale (160, 260).

5. Système selon l'une quelconque des revendications 2 à 4, dans lequel le corps (152, 252, 352) inclut une longueur à partir de l'extrémité distale (160, 260) jusqu'à l'extrémité de fixation (155, 255, 355) d'au moins 5,1 cm et une largeur maximale d'au moins 3,75 cm.

6. Système selon la revendication 2, comprenant en outre une base (170) conique ou tronconique allongée s'étroitisant au moins partiellement entre la partie cylindrique (190) et l'extrémité distale (160, 260).

7. Système selon la revendication 6, dans lequel la base (170) conique ou tronconique allongée a au moins une longueur de 3,75 cm.

8. Système selon la revendication 6 ou la revendication 7, dans lequel le corps (152, 252, 352) inclut une longueur à partir de l'extrémité distale (160, 260) jusqu'à l'extrémité de fixation (155, 255, 355) d'au moins 6,25 cm et une largeur maximale d'au moins 2,5 cm.

9. Système selon la revendication 1, dans lequel le corps (152, 252, 352) inclut une forme globalement ellipsoïdale.

10. Système selon la revendication 9, dans lequel le corps (152, 252, 352) inclut une largeur maximale d'au moins 6,25 cm.

11. Système selon la revendication 9 ou la revendication 10, dans lequel le corps (152, 252, 352) inclut une longueur de 2,5 cm à 5 cm.

12. Système selon l'une quelconque des revendications 1 à 11, dans lequel l'ouverture de réception de dispositif de collecte (180) inclut un diamètre de 1,9 cm à 3,175 cm.

13. Système selon l'une quelconque des revendications 1 à 12, dans lequel l'évidement (185) inclut une profondeur de 1,25 cm à 2,5 cm.

14. Système selon l'une quelconque des revendications 1 à 13, dans lequel l'évidement (185) inclut une partie sensiblement semi-sphérique (170).

15. Système selon l'une quelconque des revendications précédentes, et comprenant :
différents dispositifs de dimensionnement, améliorant ainsi la fixation du dispositif de collecte d'urine entre les jambes de l'individu le portant en identifiant un dispositif de dimensionnement, parmi les différents dispositifs de dimensionnement, dont le dispositif de dimensionnement identifié vient en interface ou en contact avec les jambes ou le sillon interfessier de l'individu le portant d'une manière efficace pour que le dispositif reste en place lorsque l'individu le portant est couché.
